# EUROPEAN PATENT APPLICATION

(11) **EP 4 005 626 A1**
(43) Date of publication of application: **01.06.2022**
(21) Application number: 21151733.9
(22) Date of filing: 15.01.2021
(51) Int. Cl.: A61M 37/00

(54) **WIRELESS TATTOO MACHINE CONVENIENT FOR FINGER CONTROL OPERATION**

(30) Priority: 26.11.2020 CN 202022787493 U
(71) Applicant: Chen, Xiangyu, Shanghai 200051 (CN)
(72) Inventor: Chen, Xiangyu, Shanghai 200051 (CN)
(74) Representative: Ipside

(57) **Abstract**

The present invention discloses a wireless tattoo machine convenient for finger control operation, comprising a shell (1), an electric motor (2), batteries (3), a steel rod (4) and a control circuit board (5), wherein the electric motor, the batteries, the steel rod and the control circuit board are all fixedly connected inside the shell; the electric motor is located at an upper end of the shell, and a surface of the electric motor is fixedly connected to a fixing sleeve (6); the fixing sleeve is sleeved on a surface of the shell and is slidably connected to the shell, and an output end of the electric motor is fixedly connected to an eccentric wheel (7); a surface of the eccentric wheel is provided with the movably connected steel rod, a hollow pipe (8) is arranged between a lower end of the steel rod and the shell, and the batteries and the control circuit board are arranged at an edge of the hollow pipe; and a surface of the control circuit board is sequentially provided with a charging jack (9), a pressure increasing button (10), a pressure reducing button (11), a display screen (12) and a switch button (13) from top to bottom. According to the present invention, the control circuit board integrated with the buttons is arranged on a side wall of the lower end of the shell, so that the buttons can be conveniently controlled with one hand while tattooing during a tattooing operation, and the convenience of using the tattoo machine is improved.

## Description

### Technical Field

The present invention relates to the technical field of electronic tools for tattooing, and in particular to a wireless tattoo machine convenient for finger control operation.

### Background Art

Tattoos refer to patterns or characters drawn on the skin of a person, and are one kind of human body painting art with a long history. The existing tattooing work is carried out by using a tattoo machine.

At present, control buttons of wireless tattoo machines in the market are all positioned at the top thereof, fingers cannot press the buttons in the operation process, causing that the buttons cannot be controlled with one hand while tattooing, and inconvenience is brought to an operator during use. To this end, it is necessary to design a new technical solution.

### Summary of the Invention

An object of the present invention is to provide a wireless tattoo machine convenient for finger control operation, which solves the problem in the prior art that a control button of a wireless tattoo machine cannot be pressed under the condition that the wireless tattoo machine is held with one hand during use.

In order to achieve the above object, the present invention provides the following technical solution: a wireless tattoo machine convenient for finger control operation, comprising a shell, an electric motor, batteries, a steel rod and a control circuit board, wherein the electric motor, the batteries, the steel rod and the control circuit board are all fixedly connected inside the shell; the electric motor is located at an upper end of the shell, and a surface of the electric motor is fixedly connected to a fixing sleeve; the fixing sleeve is sleeved on a surface of the shell and is slidably connected to the shell, and an output end of the electric motor is fixedly connected to an eccentric wheel; a surface of the eccentric wheel is provided with the movably connected steel rod, a hollow pipe is arranged between a lower end of the steel rod and the shell, and the batteries and the control circuit board are arranged at an edge of the hollow pipe; and a surface of the control circuit board is sequentially provided with a charging jack, a pressure increasing button, a pressure reducing button, a display screen and a switch button from top to bottom, and the control circuit board is connected to the batteries and the electric motor in series.

As an improvement of the above technical solution, there are provided two batteries which are connected in parallel, and the batteries and the control circuit board are arranged in a triangular shape.

As an improvement of the above technical solution, the lower end of the steel rod is spherical and is embedded in the hollow pipe, and the steel rod is slidably connected to the hollow pipe.

As an improvement of the above technical solution, the diameter of the middle part of the hollow pipe is smaller than the diameters of two ends thereof but larger than the diameter of the spherical lower end of the steel rod.

As an improvement of the above technical solution, a rotatably connected adjustment gear is arranged at the upper end of the shell, and the top of the shell is fixedly connected to a fixing nut.

Compared with the prior art, the present invention has the following beneficial effects:
1. According to the present invention, the control circuit board integrated with the buttons is arranged on a side wall of the lower end of the shell, so that the buttons can be conveniently controlled by one hand while tattooing during a tattooing operation, the buttons can be easily controlled by fingers during tattooing, and the convenience of using the tattoo machine is improved.
2. According to the present invention, the batteries and the control circuit board which are arranged in the triangular shape are arranged at the edge of the hollow pipe, so that the compactness of the structural design of the wireless tattoo machine is improved, the space occupied thereby is reduced, and the holding comfort of the wireless tattoo machine is improved.

### Brief Description of the Drawings

Fig. 1 is a schematic structural front view of a wireless tattoo machine convenient for finger control operation of the present invention;
Fig. 2 is a schematic structural side view of the wireless tattoo machine convenient for finger control operation of the present invention; and
Fig. 3 is a schematic structural view showing an arrangement of batteries and a control circuit board of the present invention.

In the figures: shell - 1, electric motor - 2, battery - 3, steel rod - 4, control circuit board - 5, fixing sleeve - 6, eccentric wheel - 7, hollow pipe - 8, charging jack - 9, pressure increasing button - 10, pressure reducing button - 11, display screen - 12, switch button - 13, adjustment gear - 14, and fixing nut - 15.

### Detailed Description of Embodiments

The technical solution in an embodiment of the present invention will be clearly and completely described below with reference to the accompanying drawings of the embodiment of the present invention.

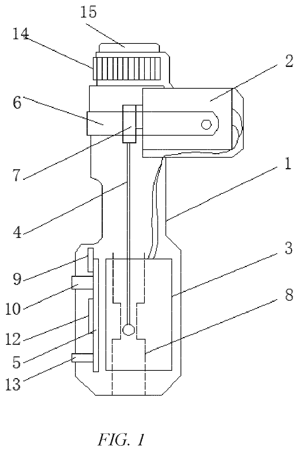

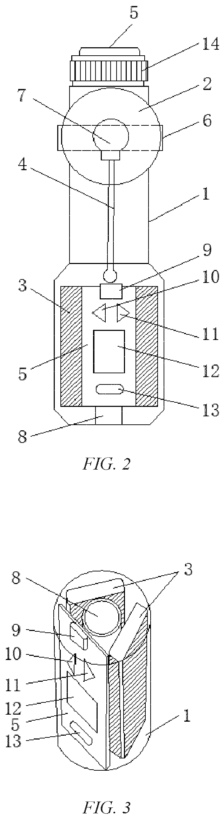

Referring to Figs. 1-3, the present invention provides a technical solution: a wireless tattoo machine convenient for finger control operation comprises a shell 1, an electric motor 2, batteries 3, a steel rod 4 and a control circuit board 5, wherein the electric motor 2, the batteries 3, the steel rod 4 and the control circuit board 5 are all fixedly connected inside the shell 1; the electric motor 2 is located at an upper end of the shell 1, and a surface of the electric motor 2 is fixedly connected to a fixing sleeve 6; the fixing sleeve 6 is sleeved on a surface of the shell 1 and is slidably connected to the shell 1, and an output end of the electric motor 2 is fixedly connected to an eccentric wheel 7; a surface of the eccentric wheel 7 is provided with the movably connected steel rod 4, a hollow pipe 8 is arranged between a lower end of the steel rod 4 and the shell 1, and the batteries 3 and the control circuit board 5 are arranged at an edge of the hollow pipe 8; and a surface of the control circuit board 5 is sequentially provided with a charging jack 9, a pressure increasing button 10, a pressure reducing button 11, a display screen 12 and a switch button 13 from top to bottom, and the control circuit board 5 is connected to the batteries 3 and the electric motor 2 in series.

As a further improvement, there are provided two batteries 3 which are connected in parallel, and the batteries 3 and the control circuit board 5 are arranged in a triangular shape. By adopting the triangular arrangement of the batteries 3 and the control circuit board 5, the utilization rate of space is improved, and the tattoo machine has a more compact structure.

As a further improvement, the lower end of the steel rod 4 is spherical and is embedded in the hollow pipe 8, and the steel rod 4 is slidably connected to the hollow pipe 8. By designing the lower end of the steel rod 4 to be spherical, it is convenient to install a needle, and the convenience of replacing a pillow is improved.

As a further improvement, the diameter of the middle of the hollow pipe 8 is smaller than the diameters of two ends thereof but larger than the diameter of the spherical lower end of the steel rod 4, and the stability of the steel rod 4 during movement is improved by providing a middle section with the smaller diameter in the middle of the hollow pipe 8.

As a specific improvement, a rotatably connected adjustment gear 14 is arranged at the upper end of the shell 1, and the top of the shell 1 is fixedly connected to a fixing nut 15. By arranging the adjustment gear 14 at the upper end of the shell 1, a movement stroke of the steel rod 4 is conveniently adjusted.

With regard to the shell 1, the electric motor 2, the batteries 3, the steel rod 4, the control circuit board 5, the fixing sleeve 6, the eccentric wheel 7, the hollow pipe 8, the charging jack 9, the pressure increasing button 10, the pressure reducing button 11, the display screen 12, the switch button 13, the adjustment gear 14 and the fixing nut 15 of the present invention, the components are all common standard components or components known to a person skilled in the art, and the structures and principles thereof would be all known to a person skilled in the art from technical manuals or by conventional experimental methods. According to the present invention, the control circuit board 5 integrated with the buttons is arranged on a side wall of the lower end of the shell 1, so that the buttons can be conveniently controlled by one hand while tattooing during a tattooing operation, the buttons can be easily controlled by fingers during tattooing, and the convenience of using the tattoo machine is improved.

When the tattoo machine of the present invention is in use, the lower end of the shell 1 is held by hand, the operation button on the surface of the control circuit board 5 is pressed, thereby adjusting the working state of the tattoo machine, the batteries 3 supply power to the electric motor 2, the electric motor 2 drives the eccentric wheel 7 to rotate, thereby driving the steel rod 4 to reciprocate up and down along the inner wall of the hollow pipe 8, and the tattooing operation is facilitated. In addition, the buttons on the surface of the control circuit board 5 may be easily pressed by fingers during the tattooing operation, so that the convenience of using the tattoo machine is improved.

Although fundamental principles and essential features of the present invention and advantages thereof are shown and described above, it would be obvious to a person skilled in the art that the present invention is not limited to the details of the foregoing exemplary embodiment, but may be implemented in other specific forms without departing from the spirit or essential features thereof. Therefore, no matter from which point of view, the embodiment should be construed as illustrative and non-restrictive, the scope of the present invention is limited by the appended claims rather than by the foregoing description, and t is thus intended that all changes that fall within the meaning and range of equivalents of the claims are included in the present invention. Any reference signs in the claims shall not be construed as limiting the claims concerned.

In addition, it should be understood that although the specification is described in terms of embodiments, not every embodiment includes only an independent technical solution, the description of the specification is for the sake of clarity only, a person skilled in the art should regard the specification as a whole. The technical solutions in the various embodiments may also be appropriately combined to form other embodiments that can be understood by a person skilled in the art.

## Claims

1. A wireless tattoo machine convenient for finger control operation, comprising a shell (1), an electric motor (2), batteries (3), a steel rod (4) and a control circuit board (5), **characterized in that** the electric motor (2), the batteries (3), the steel rod (4) and the control circuit board (5) are all fixedly connected inside the shell (1); the electric motor (2) is located at an upper end of the shell (1), and a surface of the electric motor (2) is fixedly connected to a fixing sleeve (6); the fixing sleeve (6) is sleeved on a surface of the shell (1) and is slidably connected to the shell (1), and an output end of the electric motor (2) is fixedly connected to an eccentric wheel (7); a surface of the eccentric wheel (7) is provided with the movably connected steel rod (4), a hollow pipe (8) is arranged between a lower end of the steel rod (4) and the shell (1), and the batteries (3) and the control circuit board (5) are arranged at an edge of the hollow pipe (8); and a surface of the control circuit board (5) is sequentially provided with a charging jack (9), a pressure increasing button (10), a pressure reducing button (11), a display screen (12) and a switch button (13) from top to bottom, and the control circuit board (5) is connected to the batteries (3) and the electric motor (2) in series.

2. The wireless tattoo machine convenient for finger control operation according to claim 1, **characterized in that** there are provided two batteries (3) which are connected in parallel, and the batteries (3) and the control circuit board (5) are arranged in a triangular shape.

3. The wireless tattoo machine convenient for finger control operation according to claim 1, **characterized in that** the lower end of the steel rod (4) is spherical and is embedded in the hollow pipe (8), and the steel rod (4) is slidably connected to the hollow pipe (8).

4. The wireless tattoo machine convenient for finger control operation according to claim 1, **characterized in that** the diameter of the middle of the hollow pipe (8) is smaller than the diameters of two ends thereof but larger than the diameter of the spherical lower end of the steel rod (4).

5. The wireless tattoo machine convenient for finger control operation according to claim 1, **characterized in that** a rotatably connected adjustment gear (14) is arranged at the upper end of the shell (1), and the top of the shell (1) is fixedly connected to a fixing nut (15).
